(19) Europäisches Patentamt
European Patent Office
Office européen des brevets

(11) Veröffentlichungsnummer: **0 044 418**
**B1**

(12) # EUROPÄISCHE PATENTSCHRIFT

(45) Veröffentlichungstag der Patentschrift: **22.02.84**

(51) Int. Cl.³: **C 07 D 233/96**, C 08 G 18/34 // C09D3/64

(21) Anmeldenummer: **81104770.3**

(22) Anmeldetag: **22.06.81**

(54) **Verfahren zur Herstellung von Imidazolidin-trionen.**

(30) Priorität: **21.07.80 DE 3027618**

(43) Veröffentlichungstag der Anmeldung: **27.01.82 Patentblatt 82/4**

(45) Bekanntmachung des Hinweises auf die Patenterteilung: **22.02.84 Patentblatt 84/8**

(84) Benannte Vertragsstaaten: **AT DE FR GB IT**

(56) Entgegenhaltungen: **FR - A - 2 149 713**

(73) Patentinhaber: **BAYER AG, Zentralbereich Patente, Marken und Lizenzen, D-5090 Leverkusen 1, Bayerwerk (DE)**

(72) Erfinder: **Zecher, Wilfried, Dr., Treptower Strasse 6, D-5090 Leverkusen (DE)**
Erfinder: **Merten, Rudolf, Dr., Berta-von-Suttner-Strasse 55, D-5090 Leverkusen (DE)**

EP 0 044 418 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Imidazolidin-trionen

Es ist bekannt, daß man Imidazolidintrione durch Umsetzung von 1,3-diprimären Harnstoffen mit Oxalylchlorid enthält (Chem. Ber. 46, 1387). Weitere Herstellungswege sind die Cyclisierung von Isocyanaten mit Blausäure und nachfolgende Hydrolyse der entstandenen Imino-Verbindungen (Makromol. Chem. 78, 186 und US-PS 3 661 859) und die Reaktion von Oxamidsäureestern mit Isocyanaten (DE-AS 1 770 146). Monomolekulare substituierte Imidazolin-trione können im Pharmabereich und auf dem Pflanzenschutzsektor eingesetzt werden, während Poly-imidazolidontrione insbesondere als temperaturbeständige Kunststoffe Bedeutung erlangt haben.

Es wurde nun gefunden, daß man Imidazolidin-trione in guten Ausbeuten erhält, wenn man organische Isocyanate oder entsprechende Isocyanatabspalter mit Oxalsäuremonoestern bei Temperaturen von 0—450° C, vorzugsweise von 30 bis 150° C, umsetzt.

Der Reaktionsverlauf ist überraschend, da üblicherweise aus Isocyanaten und Carbonsäuren überwiegend die entsprechenden Harnstoffe und Säureanhydride gebildet werden, die dann mit weiterem Isocyanat zu komplizierten Substanzgemischen reagieren können C. Naegelietal., Helv. Chim. Acta 17, 931 (1934). Auch zerfallen Oxalsäuremonoester bereits bei relativ niedrigen Temperaturen unter Abspaltung von Kohlendioxid, so daß eine Kondensationsreaktion nicht vorauszusehen war. Die Ausgangsmaterialien sind gut zugänglich und durch einfache Destillation zu reinigen. Auch die relativ guten Ausbeuten an Imidazolidin-trionen, die den Aufbau von Polymeren ermöglichen, waren bei der erfindungsgemäßen Reaktion, die über mehrere Stufen verläuft, nicht zu erwarten. Als Nebenprodukte werden bei der Polykondensation lediglich Kohlendioxid und Alkohol frei.

Die erfindungsgemäß als Ausgangsmaterialien verwendeten Oxalsäuremonoester sind z. B. aus Oxalsäure durch partielle Veresterung oder aus Oxalsäurediestern durch partielle Hydrolyse zugänglich und können in Substanz eingesetzt oder auch im Reaktionsmedium aus den Komponenten hergestellt werden. Diese Verbindungen entsprechen der allgemeinen Formel

$$R_1(OOCCOOH)_n$$

in der $R_1$ einen n-wertigen, gegebenenfalls substituierten aliphatischen Rest mit $C_1-C_{22}$, bevorzugt $C_1-C_6$, aliphatisch-aromatischen Rest mit $C_7-C_{20}$, bevorzugt $C_7-C_{10}$, oder aromatischen Rest mit $C_6-C_{10}$, heterocyclischen Rest mit $C_3-C_{10}$ C-Ringatomen und 1—3 Heteroatomen, wie N, S und/oder O im Ring oder Polyether-, Polyester- oder Polyurethanrest und n eine ganze Zahl von 1—3, vorzugsweise 1 bedeuten. Der Rest $R_1$ leitet sich vorzugsweise von Methan, Ethan, n-, iso-, tert.-Butan, Hexan, Eicosan, Propen, Butin, Cyclohexan, Benzol, Naphthalin, Diphenylmethan, — oder kernsubstituiertem Toluol und Xylol ab. Der Rest $R_1$ kann einfach oder mehrfach mit Halogen oder Alkyl $C_1-C_6$ oder Aryl-Resten $C_6-C_{10}$, Carbonsäure- oder Carbonsäureestergruppen substituiert sein.

Besonders bevorzugt werden die Monoester der Oxalsäure mit monofunktionellen aliphatischen Alkoholen mit $C_1-C_6$ eingesetzt.

Als organische Monoisocyanate im Sinne der Erfindung werden aliphatische und aromatische, gegebenenfalls durch Heteroatome substituierte Verbindungen mit einer NCO-Gruppe im Molekül eingesetzt, z. B. Alkylisocyanate wie Ethyl-, Methyl-, Butyl-, Dodecyl- und Stearylisocyanat, aromatische, gegebenenfalls substituierte Monoisocyanate wie Phenyl-, Tolyl-, Isopropyl-, Nonylisocyanat-, Nitro-, Alkoxy-, Aroxy-, Chlor-, Dichlor-, Trichlor-, Tetra-, Pentachlor-, Benzyl-, Brom-phenyl-isocyanat oder Isocyanatobenzoesäureester, Phthalsäureester, Isophthalsäureester, Isocyanatobenzonitril, cycloaliphatische Isocyanate wie Cyclohexylisocyanat und ungesättigte Isocyanate wie Allyl-, Oleyl-, Cyclohexenyl-isocyanat.

Als erfindungsgemäß einzusetzende Ausgangskomponenten kommen ferner aliphatische, cycloaliphatische, araliphatische, aromatische und heterocyclische Polyisocyanate, vorzugsweise Diisocyanate, in Betracht (vgl. Annalen, 562, Seite 75 bis 136), beispielsweise Ethylendiisocyanat, 1,4-Tetramethylendiisocyanat, 1,6-Hexamethylen-diisocyanat, 1,12-Dodecandiisocyanat, Cyclobutan-1,3-diisocyanat, Cyclohexan-1,3- und -1,4-diisocyanat sowie beiliebige Gemische dieser Isomeren, 1-Isocyanato-3,3,5-trimethyl-5-isocyanato-methyl-cyclohexan (DE-AS 1 202 785), 2,4- und 2,6-Hexahydrotoluylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4''-Triisocyanat, Polyphenyl-polymethylen-polyisocyanate, wie sie durch Anilin-Formaldehyd-Kondensation und anschließende Phosgenierung erhalten und z. B. in den britischen Patentschriften 874 430 und 848 671 beschrieben werden, perchlorierte Arylpolyisocyanate, wie sie z. B. in der deutschen Auslegeschrift 1 157 601 beschrieben werden, Carbodiimidgruppen aufweisende Polyisocyanate, wie sie in der deutschen Patentschrift 1 092 007 beschrieben werden, Diisocyanate, wie sie in der US-Patentschrift 3 492 330 beschrieben werden, Allophanatgruppen aufweisende Polyisocyanate, wie sie z. B. in der britischen Patentschrift 99 800, der belgischen 761 626 und der

veröffentlichten holländischen Patentanmeldung 7 102 524 beschrieben werden, Isocyanuratgruppen aufweisende Polyisocyanate, wie sie z. B. in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z. B. in der belgischen Patentschrift 752 261 oder in der US-Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z. B. in der deutschen Patentschrift 1 101 394 in der britischen Patentschrift 889 050 und in der französischen Patentschrift 7 017 514 beschrieben werden, durch Telomerisationsreaktion hergestellte Polyisocyanate, wie sie z. B. in der belgischen Patentschrift 723 640 beschrieben werden, Estergruppen aufweisende Polyisocyanate, wie sie z. B. in den britischen Patentschriften 956 474 und 1 072 956, in der US-Patentschrift 3 567 763 und in der deutschen Patentschrift 1 231 688 genannt werden. Umsetzungsprodukte der obengenannten Isocyanate mit Acetylen gemäß der deutschen Patentschrift 1 072 358.

Es ist auch möglich, die bei der technischen Isocyanatherstellung anfallenden Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Erfindungsgemäß eigenen sich Mono- bzw. Polyisocyanate der allgemeinen Formel

$$R_2(-NCO)_z$$

in denen $R_2$ für einen, gegebenenfalls mit Halogen, Alkyl- und/oder Arylgruppen substituierten aliphatischen Rest mit 1—20 C-Atomen, einen aromatischen Rest mit 6—12 C-Atomen, einen cycloaliphatischen Rest mit 5—12 C-Atomen, einen aliphatisch-aromatischen Rest mit 7—20 C-Atomen und einen Heteroatome wie N, O oder S im Ring enthaltenden aromatischen oder cycloaliphatischen Rest mit 5—12 C-Ringatomen, steht. Bevorzugt sind aliphatische Reste mit 2—12 C-Atomen, oder ein Arylrest wie Phenyl, Tolyl, Naphthyl, Diphenylmethan und Diphenyletherreste. z ist eine ganze Zahl von 1—4, vorzugsweise 1—3, besonders bevorzugt 2.

Bevorzugt verwendet werden die technisch leicht zugänglichen Gemische aus Toluylen-diisocyanaten, m-Phenylendiisocyanat, Phenylisocyanat und seine Substitutionsprodukte, Methylisocyanat, sowie phosgenierte Kondensate aus Anilin und Formaldehyd mit Polyphenylenmethylen-struktur und die symmetrische Verbindungen 4,4'-Diisocyanato-diphenylmethan, 4,4'-Diisocyanatodiphenylether, p-Phelyndiisocyanat, 4,4'-Diisocyanato-diphenyl-dimethylmethan, analog hydroaromatische Diisocyanate, sowie aliphatische Diisocyanate mit 2—12 C-Atomen wie Hexamethylendiisocyanat und von Isophoron abgeleitete Diisocyanate.

Die Isocyanate können in freier Form, ferner zum Teil oder vollständig auch in Form ihrer, beim Umsatz mit reaktiven Wasserstoff enthaltenden Verbindungen, zugänglichen und unter den Reaktionsbedingungen als Abspalter reagierenden Derivate eingesetzt werden.

Erfindungsgemäß werden als Abspalter die als Lactamen, z. B. Caprolactam, zugänglichen Acyl-Harnstoffen und die aus aromatischen und aliphatischen Mono- und Polyhydroxy-Verbindungen erhaltenen Carbamidsäureester, die den allgemeinen Formeln

$$R_2\left(-NH-\underset{\underset{O}{\|}}{C}-O-A\right)_z \quad \text{bzw.} \quad \left[-\underset{\underset{O}{\|}}{C}-NH-R_2-NH-\underset{\underset{O}{\|}}{C}-O-B-O-\right]_n$$

entsprechen, eingesetzt, wobei $R_2$ und z die oben angegebene Bedeutung haben und A der organische Rest einer Monohydroxyverbindung bzw. B der organische Rest einer bis- oder trisfunktionellen Hydroxyverbindung die sich jeweils von einem aliphatischen Rest mit 1—10 C-Atomen, einem cycloaliphatischen Rest mit 5—10 C-Atomen, einem aliphatisch-aromatischen Rest mit 7—12 C-Atomen und einem aromatischem Rest mit 6—12 C-Atomen ableiten und n für eine ganze Zahl von 1—100 steht.

Als Beispiele seien die Carbamidsäureester aus Phenol, isomeren Kresolen, deren technischen Gemischen und ähnlichen aromatischen Hydroxylverbindungen, aliphatische Monoalkohole wie Methanol, Ethanol, Propanol, Isopropanol, Butanol, Isobutanol, Diethylenglykolmonomethylether, Cyclohexanol, Benzylalkohol und aliphatische Di- oder Polyole wie Ethylenglykol und Trimethylolpropan aufgeführt.

Die Urethane können als solche eingesetzt oder erst in situ durch Umsetzung mit Alkoholen erzeugt werden.

Durch das nachfolgende Reaktionsschema soll die erfindungsgemäße Reaktion erläutert werden:

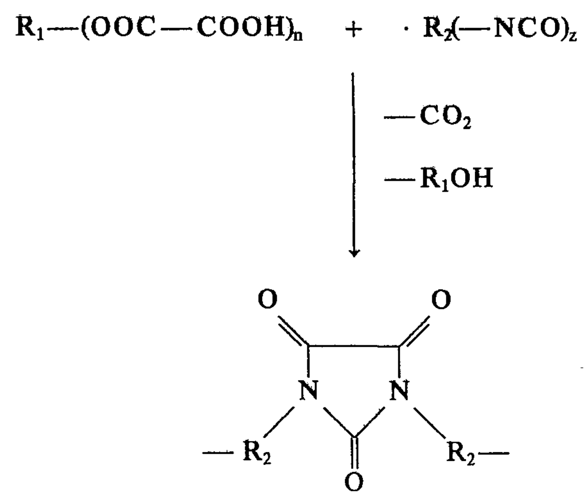


I

wobei die Reste $R_1$ und $R_2$ die oben angegebene Bedeutung haben und für $z=1$ eine monomolekulare und für $z>1$ eine höhermolekulare Verbindung mit der wiederkehrenden Struktureinheit (I) entsteht, wobei die Imidazolidintrion-Ringe über die Reste $R_2$ verknüpft sind.

Die erfindungsgemäßen Imidazolidintrione können durch IR-Spektren, in denen die charakteristischen Banden für Imidazolidintrione auftreten, identifiziert werden. Die höhermolekularen Imidazolidintrione zeigen eine Lösungsviskosität von 100 bis 200 000 mPa · s, vorzugsweise von 1000 bis 50 000, die an einer 30gew.-%igen Lösung in z. B. N-Methylpyrrolidon bei 25° C bestimmt wird.

Die erfindungsgemäße Reaktion kann in Lösungsmitteln, die unter den Reaktionsbedingungen nicht reagieren oder nur lockere Additionsverbindungen bilden, oder auch in einem Überschuß einer der Reaktionskomponenten ausgeführt werden. Geeignete Lösungsmittel sind: (Halogen)-Kohlenwasserstoffe, Phenole, Alkohole, Ester, Lactone, Ketone, Ether, substituierte Amide, Nitrile, Phosphorsäureamide, Sulfoxide, und Sulfone, beispielsweise Xylole, o-Dichlorbenzol, Phenol, Kresole, Benzoesäurealkylester, Phthalsäuredimethylester, Butyrolacton, Caprolacton, Acetophenon, Cyclohexanon, Benzylalkohol, Ethylenglykol, Glykolmonomethyletheracetat, Diethylenglykolmonoethylether, Diethylenglykoldimethylether, Dimethylformamid, N-Methylpyrrolidon, Caprolactam, Benzonitril, Hexamethylphosphorsäuretriamid, Dimethylsulfoxid, Tetramethylensulfon und deren Gemische.

Das erfindungsgemäße Verfahren wird vorzugsweise so durchgeführt, daß die Reaktionskomponenten mit oder ohne Lösungsmittel einige Minuten bis zu mehreren Stunden bei Temperaturen von etwa 0–450° C, vorzugsweise 30–150° C gehalten werden. Der Verlauf der Reaktion läßt sich über die Gasentwicklung, die Abspaltung des Alkohols und die IR-Spektren verfolgen. Es ist zuweilen vorteilhaft, die Reaktion in mehreren Stufen durchzuführen oder die einzelnen Komponenten in unterschiedlicher Reihenfolge oder bei verschiedenen Temperaturen zuzugeben. Insbesondere bei der Herstellung von Polymeren kann auch in einer ersten Stufe, z. B. in einem Lösungsmittel, ein Kondensationsprodukt hergestellt werden, das dann bei höheren Temperaturen unter Kettenverlängerung oder Vernetzung und gegebenenfalls Verdampfen des Lösungsmittels in das hochmolekulare Reaktionsprodukt, z. B. einen Lackfilm, übergeführt wird. Bei der Verwendung in Lacken können diese auch aus Schmelzen oder wäßrigen Dispersionen appliziert werden.

Im allgemeinen werden äquivalente Mengen Oxalsäuremonoester und Isocyanatgruppen eingesetzt. Es sind aber auch sehr weitgehende Abweichungen von diesen Mengenverhältnissen möglich. Aus Monoestern und monofunktionellen Isocyanaten werden monomolekulare Imidazolidintrione, aus Monoestern und Polyisocyanaten in Abhängigkeit von den stöchiometrischen Verhältnissen höhermolekularer Imidazolidintrione oder Oligomere, z. B. Imidazolidin-isocyanate oder -urethane, erhalten.

Eine weitere Ausführungsform der erfindungsgemäßen Reaktion für den Polymer-Bereich besteht darin, daß die Kondensation in Gegenwart von Poly-alkoholen, -aminen, -carbonsäuren, -carbonsäureanhydriden und/oder überschüssigen -isocyanaten oder deren Kombination wie Aminoalkoholen oder Aminocarbonsäuren durchgeführt wird. So werden mit Terephthalsäure und Ethylenglykol Polyimidazolidintrion-ester und mit Trimellitsäureanhydrid und Diisocyanaten Poly-imidazolidintrion-amidimide erhalten.

Die erfindungsgemäße Reaktion kann durch übliche Isocyanat-Katalysatoren beschleunigt werden, z. B. durch Amine wie Triethylamin, 1,4-Diazobicyclo-(2,2,2)-octan, N-Ethyl-morpholin, N-Methylimidazol, 4-Dimethylaminopyridin und 4-(1-Pyrolidino)-pyridin, sowie durch organische und anorganische Metallverbindungen, insbesondere von Eisen, Blei, Zink, Zinn, Kupfer, Kobalt und Titan, wie Eisen(III)-chlorid, Kobaltacetat, Bleioxid, Bleiacetat, Zinnoctoat, Dibutyl-zinn-dilaurat. Kupferacetylacetonat, Titantetrabutylat, Alkali-phenolate und Natriumcyanid und durch Phosphorverbindungen wie Trialkylphosphin und Methylphospholinoxid und Hydroxy-Aromaten wie Phenol, Hydrochinon und Resorcin.

Die nach dem erfindungsgemäßen Verfahren herstellbaren monomolekularen Imidazolidintrione zeigen Wirkungen auf dem pharmazeutischen und dem Pflanzenschutzsektor. Die erfindungsgemäßen Polyimidazolidintrione zeichnen sich durch besondere Temperaturbeständigkeit aus und sind geeignet zur Verwendung als Kleber, Lacke, Folien und Formkörper. Ihre Eigenschaften können für die verschiedenen Einsatzgebiete durch Zusatz von Füllstoffen, Pigmenten und nieder- und hochmolekularen Komponenten, z. B. zur Herstellung von Lacken und Folien durch Abmischen mit Polyestern und Polycarbamidestern, in weiten Grenzen variiert werden.

Beispiel 1

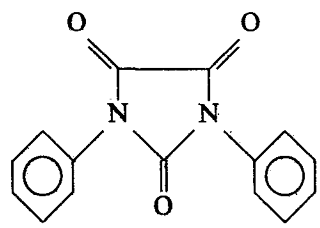

59 g Oxalsäuremonoethylester, 179 g Phenylisocyanat und 0,2 g Triethylendiamin werden in 240 g N-Methylpyrrolidon eingetragen. Dann wird unter Rühren 4 Stunden auf 80° C, 2 Stunden auf 100° C und 2 Stunden auf 120° C erhitzt. Die Kondensation und anschließende Cyclisierung erfolgt unter Abspaltung von Kohlendioxid und Ethanol. Das Reaktionsprodukt wird mit Wasser gefällt und die Fällung zur Abtrennung von N-Phenylcarbamidsäureethylester mit Methanol verrührt. Man erhält als Rückstand beim Absaugen 102 g (78% d. Th.) 1,3-Diphenylimidazolidin-2,4,5-trion vom Schmp. 190 – 197° C. Zur Reindarstellung wird mit Acetonitril aufgenommen, filtriert, eingedampft und der Rückstand aus Toluol umkristallisiert. Man erhält die reine Verbindung in farblosen Polyedern vom Schmp. 205 – 206° C und einer charakteristischen Bande im IR-Spektrum bei 1750 $cm^{-1}$.

$C_{15}H_{10}N_2O_3$ (266)

| | | | |
|---|---|---|---|
| ber. | C 67,7H | 3,8N | 10,5% |
| gef. | C 67,6 | H 3,8 | N 10,7% |

Beispiel 2

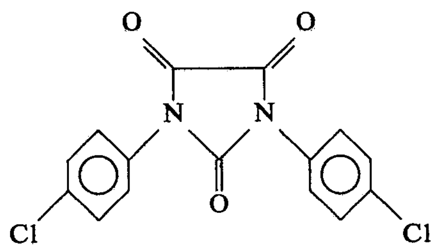

In 200 g Butyrolacton wurden 26 g Oxalsäuremonomethylester und 77 g 4-Chlorphenylisocyanat gelöst. Dann wird die Temperatur langsam auf 120° C gesteigert und 6 Stunden bei 120° C und 2 Stunden bei 140° C gerührt. Die Reaktion zum Imidazolidin-trion erfolgt unter Abspaltung von Kohlendioxid und Methanol. Nach Beendigung der Reaktion wird das Reaktionsgemisch mit Wasser versetzt und die Fällung in Aceton aufgenommen, filtriert und das Filtrat eingedampft. Der Eindampfrückstand ergibt bei der Umkristallisation aus Acetonitril das 1,3-Bis-(4-chlorphenyl)-imidazolidin-trion in farblosen Polyedern vom Schmp. 257 – 258° C und einer Bande bei 1740 $cm^{-1}$.

$C_{15}H_8Cl_2N_2O_3$ (335)

| | | | |
|---|---|---|---|
| ber. | C 53,7 | H 2,4 | N 8,4% |
| gef. | C 53,8 | H 2,4 | N 8,6% |

## Beispiel 3

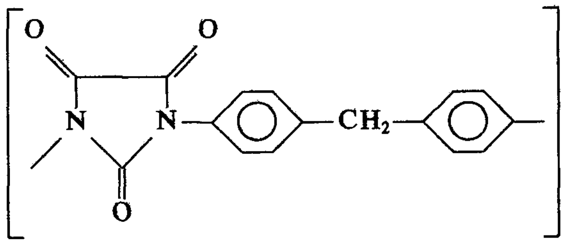

59 g Oxalsäuremonoethylester und 130 g 4,4'-Diisocyanatodiphenylmethan werden in 320 g N-Methylpyrrolidon eingetragen. Dann wird 4 Stunden bei 80° C gerührt und danach die Temperatur in jeweils zwei Stunden um 20° C bis auf 200° C gesteigert. Anschließend wird noch 4 Stunden bei dieser Temperatur gerührt. Man erhält das Polyimidazolidin-trion mit der angegebenen, wiederkehrenden Struktureinheit als braune klare Lösung mit einer Viskosität von 560 mPas und einer für Imidazolidin-trione charakteristischen Bande bei 1750 $cm^{-1}$.

Eine Probe der Lösung wird auf eine Glasplatte aufgestrichen und in jeweils 15 Min. bei 200 und 300° C zu einer klaren elastischen Folie eingebrannt. Aus einer weiteren Probe wird das Polyimidazolidintrion mit Wasser als gelbbraunes Pulver ausgefällt.

$(C_{16}H_{10}N_2O_3)_n$ (278)n

| | | | |
|---|---|---|---|
| ber. | C 69,1 | H 3,6 | N 10,1% |
| gef. | C 69,3 | H 4,0 | N 10,5% |

## Beispiel 4

Die Reaktion wird entsprechend Beispiel 3 in 320 g eines technischen Kresolgemisches als Lösungsmittel ausgeführt. Das Poly-imidazolidin-trion wird als braune viskose Lösung erhalten, die auf einer Spiegelfolie in jeweils 15 Min. bei 200 und 300° C zu einem klaren elastischen Lackfilm eingebrannt wird. Das IR-Spektrum enthält bei 1750 $cm^{-1}$ die für Imidazolidintrione charakteristische Bande.

## Beispiel 5

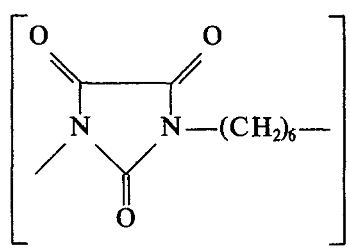

In 120 g Dimethylacetamid werden 26,0 g Oxalsäuremonomethylester und 43,7 g Hexamethylendiisocyanat gelöst, dann wird jeweils 4 Stunden auf 80, 120, 130, 140 und 150° C erhitzt. Man erhält das Polyimidazolidintrion mit der angegebenen wiederkehrenden Struktureinheit als braune viskose Lösung, von der eine Probe auf einem Spiegelblech in jeweils 15 Min. bei 200 und 300° C zu einem klaren harten Lackfgilm eingebrannt wird. Das IR-Spektrum enthält eine für Imidazolidintrion charakteristische Bande bei 1750 $cm^{-1}$.

## Beispiel 6

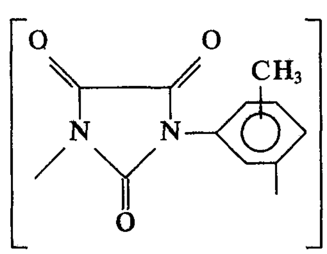

61 g Oxalsäuremonoisopropylester und 90,5 g eines technischen Gemisches aus 80% 2,4- und 20% 2,6-Toluylendiisocyanat werden in 260 g Butyrolacton gelöst. Dann wird jeweils 4 Stunden bei 80, 120,

140 und 150°C gerührt, 0,3 g Triethylendiamin zugegeben und zur Beendigung der Reaktion noch 10 Stunden auf 150°C erhitzt. Man erhält das Polyimidazolidintrion mit der angegebenen, wiederkehrenden Struktureinheit als braune viskose Lösung.

Eine Probe der Lösung wird auf einem Spiegelblech bei 200 und 300°C zu einem klaren harten Lackfilm eingebrannt, der im IR-Spektrum eine charakteristische Bande bei 1755 $cm^{-1}$ zeigt.

Aus einer weiteren Probe wird das Polyimidazolidintrion als gelbes Pulver mit einer Bande bei 1755 $cm^{-1}$ gefällt.

### Beispiel 7

Poly-amid-imid-triazolidintrion

29,6 g Oxalsäuremonoethylester, 48,0 g Trimellitsäureanhydrid und 130 g 4,4'-Diisocyanato-diphenylmethan werden in 370 g N-Methylpyrrolidin eingetragen. Dann wird unter Rühren jeweils 4 Stunden auf 80, 120, 140 und 150°C erhitzt. Die Kondensation erfolgt unter Abspaltung von Kohlendioxid und Ethanol. Man erhält das Poly-amid-imid-triazolidin-trion als klare braune Lösung mit einer Viskosität $\eta 25 = 360$ mPas.

Aus einer Probe dieser Lösung wird das Polykondensat als gelbes Pulver gefällt, das bei 1670 $cm^{-1}$ für Amide, bei 1750 $cm^{-1}$ für Imidazolidintrione und bei 1720 und 1780 $cm^{-1}$ für Imide charakteristische Banden zeigt.

Eine weitere Probe der Lacklösung wird auf einer Glasplatte bei 200 und 300°C zu einem klaren elastischen Lackfilm eingebrannt. Anstelle von N-Methylpyrrolidon kann auch ein Gemisch aus Kresol und Phenol als Lösungsmittel verwendet werden.

**Patentansprüche**

1. Verfahren zur Herstellung von Imidazolidintrionen, dadurch gekennzeichnet, daß organische Mono- bzw. Polyisocyanate der allgemeinen Formel

$$R_2(-NCO)_z$$

in denen

$R_2$ für einen gegebenenfalls mit Halogen, Alkyl- und/oder Arylgruppen substituierten aliphatischen Rest mit 1–20 C-Atomen, einen aromatischen Rest mit 6–10 C-Atomen, einen cycloaliphatischen Rest mit 5–12 C-Atomen, einen aliphatisch-aromatischen Rest mit 7–20 C-Atomen und einen Heteroatome wie N, O oder S im Ring enthaltenden aromatischen oder cycloaliphatischen Rest mit 5–12 C-Ringatomen,

z für eine ganze Zahl von 1 bis 4, steht,

oder entsprechende Isocyanatabspalter der allgemeinen Formeln

$$R_2\left(-NH-\underset{\underset{O}{\|}}{C}-O-A\right)_z \quad \text{bzw.} \quad \left[-\underset{\underset{O}{\|}}{C}-NH-R_2-NH-\underset{\underset{O}{\|}}{C}-O-B-O-\right]_n$$

in welchen

$R_2$ und z die oben angegebene Bedeutung haben und

A der organische Rest einer Monohydroxyverbindung, bzw.

B der organische Rest einer bis- oder trisfunktionellen Hydroxyverbindung, die sich jeweils von einem aliphatischen Rest mit 1–10 C-Atomen, einem cycloaliphatischen Rest mit 5–10 C-Atomen, einem aliphatisch-aromatischen Rest mit 7–12 C-Atomen und einem aromatischen Rest mit 6–12 C-Atomen ableiten, und

n für eine ganze Zahl von 1 bis 100 steht

mit Oxalsäuremonoestern der allgemeinen Formel

$$R_1(OOCCOOH)_n$$

in der

$R_1$ einen n-wertigen, gegebenenfalls einfach oder mehrfach mit Halogen, $C_1-C_6$-Alkyl, $C_6-C_{10}$-Aryl, Carbonsäure oder Carbonsäureestergruppen substituierten aliphatischen Rest mit $C_1-C_{22}$, aliphatisch-aromatischen Rest mit $C_7-C_{20}$, oder aromatischen Rest mit $C_6-C_{10}$, heterocyclischen Rest mit $C_3-C_{10}$ C-Ringatomen und 1–3 Heteroatomen, wie N, S und/oder O im Ring oder Polyether, Polyester- oder Polyurethanrest und

n eine ganze Zahl von 1 bis 3 bedeuten,

gegebenenfalls in Anwesenheit von weiteren kondensationsfähigen Polymerverbindungen und/oder Katalysatoren und/oder Lösungsmitteln bei Temperaturen von 0–450° C umgesetzt werden.

2. Verfahren nach Anspruch 1, dadurch gekennzeichnet, daß in der allgemeinen Formel $R_1$ sich von Methan, Ethan, n-, iso-, tert.-Butan, Hexan, Eicosan, Propen, Butin, Cyclohexan, Benzol, Naphthalin, Diphenylmethan, Toluol oder Xylol ableitet und n für 1 steht.

3. Verfahren nach Ansprüchen 1 und 2, dadurch gekennzeichnet, daß als Monoester $C_1-C_6$-Alkyl-Monoester der Oxalsäure verwendet werden.

4. Verfahren nach Ansprüchen 1 bis 3, dadurch gekennzeichnet, daß als Isocyanate bzw. -abspalter Polyisocyanat(abspalter) verwendet werden.

5. Verfahren nach Ansprüchen 1 bis 4, dadurch gekennzeichnet, daß als Isocyanate bzw. -abspalter Diisocyanat(abspalter) verwendet werden.

**Claims**

1. Process for the production of imidazolidine triones, characterised in that organic mono- or poly-isocyanates of the general formula

$$R_2(-NCO)_z$$

in which

$R_2$ represents an aliphatic radical which has 1–20 C atoms and is optionally substituted by halogen, alkyl and/or aryl groups, an aromatic radical with 6–10 C atoms, a cycloaliphatic radical with 5–12 C atoms, an aliphatic-aromatic radical with 7–20 C atoms and an aromatic or cycloaliphatic radical which has 5–12 C ring atoms and contains hetero atoms such as N, O or S in the ring, and

Z represents an integer from 1 to 4,

or corresponding isocyanate donors of the general formulae

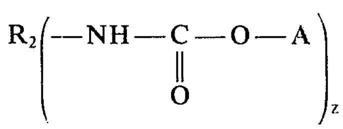 or 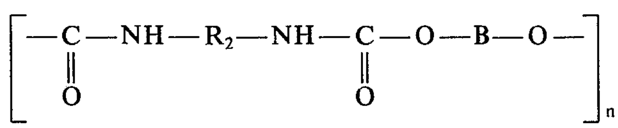

in which

$R_2$ and z have the meaning indicated above and

A denotes the organic radical of a monohydroxy compound, or

B denotes the organic radical of a bis- or tris-functional hydroxy compound, which are in each case derived from an aliphatic radical with 1–10 C atoms, a cycloaliphatic radical with 5–10 C atoms, an aliphatic-aromatic radical with 7–12 C atoms and an aromatic radical with 6–12 atoms, and

n represents an integer from 1 to 100,

are reacted with oxalic acid mono esters of the general formula

$$R_1(OOCCOOH)_n$$

in which

$R_1$ denotes an n-valent aliphatic radical with $C_1-C_{22}$ which is optionally mono- or poly-substituted by halogen, $C_1-C_6$-alkyl, $C_6-C_{10}$-aryl, carboxylic acid or carboxylic acid ester groups, an aliphatic-aromatic radical with $C_7-C_{20}$, or an aromatic radical with $C_6-C_{10}$, heterocyclic radical with $C_3-C_{10}$ C ring atoms and 1–3 hetero atoms, such as N, S and/or O in the ring or a polyether, polyester or polyurethane radical and

n denotes an integer from 1 to 3,

optionally in the presence of other polymer compounds capable of being cocondensed and/or catalysts and/or solvents, at temperatures of 0–450° C.

2. Process according to Claim 1, characterised in that in the general formula $R_1$ is derived from methane, ethane, n-, iso- or tert.-butane, hexane, eicosane, propene, butine, cyclohexane, benzene, naphthalene, diphenylmethane, toluene or xylene and n represents 1.

3. Process according to Claims 1 and 2, characterised in that the monoesters used are $C_1-C_6$-alkyl-monoesters of oxalic acid.

4. Process according to Claims 1 to 3, characterised in that the isocyanates or isocyanate donors used are polyisocyanate (donors).

5. Process according to Claims 1 to 4, characterised in that the isocyanates or isocyanate donors used are diisocyanate (donors).

**Revendications**

1. Procédé de préparation d'imidazolidinetriones, caractérisé en ce qu'on fait réagir, éventuellement en présence d'autres composés polymères capables d'une condensation et/ou de catalyseurs et/ou de solvants, à des températures de 0 à 450° C, des mono- ou polyisocyanates organiques de formule générale:

$$R_2(-NCO)_z$$

dans laquelle

$R_2$ est un radical aliphatique comportant 1 à 20 atomes de carbone et éventuellement substitué par de l'halogène ou par des groupes alkyles et/ou aryles, un radical aromatique ayant 6 à 10 atomes de carbone, un radical cycloaliphatique ayant 5 à 12 atomes de carbone un radical aliphatique-aromatique comportant 7 à 20 atomes de carbone et un radical aromatique ou cycloaliphatique comportant 5 à 12 atomes de carbone et contenant dans le noyau un hétéroatome vomme N, O ou S;

z est un nombre entier valant 1 à 4,

ou de composés correspondants pouvant céder de l'isocyanate et répondant aux formules générales:

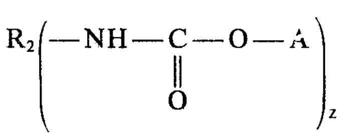

ou

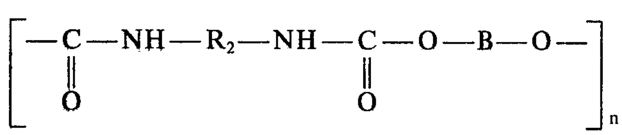

[dans lesquelles

$R_2$ et z ont le sens indiqué ci-dessus et

A représente le radical organique d'un composé monohydroxylé, ou

B représente le radical organique d'un composé hydroxylé bis-ou trisfonctionnel, qui provient à chaque fois d'un radical aliphatique ayant 1 à 10 atomes de carbone, d'un radical cycloaliphatique ayant 5 à 10 atomes de carbone, d'un radical aliphatique-aromatique ayant 7 à 12 atomes de carbone et d'un radical aromatique ayant 6 à 12 atomes de carbone, et

n est un nombre entier valant 1 à 100]

avec des monoesters de l'acide oxalique, de formule générale:

$$R_1(OOCCOOH)_n$$

[dans laquelle

$R_1$ est un radical aliphatique de valence n ayant 1 à 22 atomes de carbone (éventuellement substituée une ou plusieurs fois par de l'halogène, par un groupe alkyle en $C_1-C_6$, aryle en $C_6-C_{10}$, acide carboxylique ou ester d'acide carboxylique), un radical aliphatique-aromatique en $C_7-C_{20}$ ou un radical aromatique comportant 6 à 10 atomes de carbone, un radical hétérocycliqué comportant 3 à 10 atomes de carbone dans le noyau et 1 à 3 hétéroatomes comme N, S et/ou O dans le noyau ou un radical polyéther, polyester ou polyuréthane, et

n est un nombre entier valant 1 à 3].

2. Procédé selon la revendication 1, caractérisé en ce que, dans la formule générale, $R_1$ dérive du méthane, de l'éthane, du n-butane, de l'isobutane, du tertiobutane, de l'hexane, de l'eicosane, du

propène, du butyne, du cyclohexane, du benzène, du naphthalène, du diphénylméthane, du toluène ou du xylène, et n vaut 1.

3. Procédé selon les revendications 1 et 2, caractérisé en ce que l'on utilise comme monoester un monoester alkylique en $C_1-C_6$ de l'acide oxalique.

4. Procédé selon les revendications 1 à 3, caractérisé en ce que l'on utilise comme isocyanates ou comme composés cédant des isocyanates du polyisocyanate ou un composé cédant du polyisocyanate.

5. Procédé selon les revendications 1 à 4, caractérisé en ce que l'on utilise comme isocyanates ou composés cédant des isocyanates du diisocyanate ou un composé qui en cède.